# EUROPÄISCHE PATENTSCHRIFT

(11) **EP 3 263 548 B1**
(45) Veröffentlichungstag und Bekanntmachung des Hinweises auf die Patenterteilung: **31.07.2019**
(21) Anmeldenummer: 17000967.4
(22) Anmeldetag: 08.06.2017
(51) Int. Cl.: C07C 257/14, C07C 209/52, C07C 211/15

(54) **VERFAHREN ZUR SYNTHESE VON 1,1-DIAMINO-2,2-DINITROETHYLEN (FOX-7) ODER EINES SALZES DAVON**
METHOD FOR THE SYNTHESIS OF 1,1-DIAMINO-2,2-DINITROETHYLENE (FOX-7) OR A SALT THEREOF
PROCÉDÉ DE SYNTHÈSE DE 1,1-DIAMINO-2,2-DINITROÉTHYLE (FOX-7) OU SES SELS

(30) Priorität: 29.06.2016 DE 102016007866
(43) Veröffentlichungstag der Anmeldung: 03.01.2018
(73) Patentinhaber: Diehl Defence GmbH & Co. KG, 88662 ÜBERLINGEN (DE)
(72) Erfinder: Pham-Schönwetter, Oliver, DE - 91207 Lauf (DE); Hahma, Arno, DE - 91239 Henfenfeld (DE); Donner, Björn, DE - 91325 Adelsdorf (DE)
(74) Vertreter: Diehl Patentabteilung

(56) Entgegenhaltungen:
- NIKOLAJ V. LATYPOV ET AL: "On the Synthesis of 1,1-Diamino-2,2-dinitroethene (FOX-7) by Nitration of 4,6-Dihydroxy-2-methylpyrimidine", ORGANIC PROCESS RESEARCH AND DEVELOPMENT, Bd. 11, Nr. 1, 1. Januar 2007 (2007-01-01) , Seiten 56-59, XP055393872, US ISSN: 1083-6160, DOI: 10.1021/op068010t

## Beschreibung

Die Erfindung betrifft die Synthese von 1,1-Diamino-2,2-dinitroethylen (FOX-7) oder eines Salzes von 1,1-Diamino-2,2-dinitroethylen. Zur Herstellung von FOX-7 sind verschiedene Methoden bekannt:

### 1. Nitrierung eines Stickstoffheterozyklus mit anschließender Hydrolyse zum Endprodukt

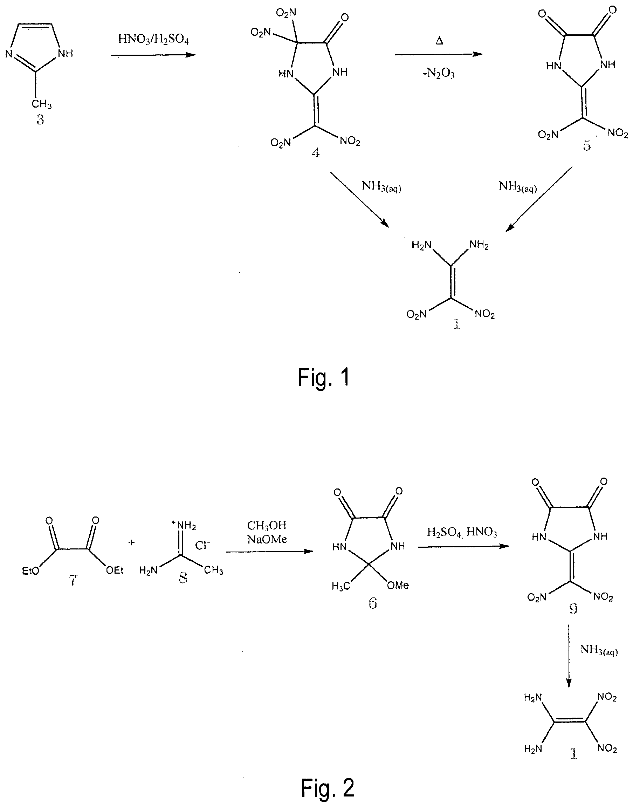

Die Synthese ist in Fig. 1 schematisch dargestellt. Bei dem Stickstoffheterozyklus kann es sich beispielsweise um Methylimidazol (3) handeln. Bei dessen Nitrierung mittels HNO₃/H₂SO₄ kommt es durch eine Nitrierung der Methylgruppe und einer Oxidation der beiden nicht an der Bindung der Methylgruppe beteiligten Kohlenstoffatome des Imidazolrings zur Bildung einer Zwischenverbindung (4). Diese Zwischenverbindung (4) zerfällt bei Raumtemperatur teilweise in eine weitere Zwischenverbindung (5), wobei aus beiden Zwischenverbindungen (4, 5) durch Hydrolyse in flüssigem Ammoniak FOX-7 (1) entsteht. Problematisch bei dieser Synthese ist, dass die weitere Zwischenverbindung (5) sehr empfindlich ist und leicht explodiert. Weiterhin ist die Nitrierungsreaktion verhältnismäßig ineffizient. Dies führt zu einer geringen Gesamtausbeute von üblicherweise höchstens 13%.

### 2. Nitrierung von 2-Methyl-2-Methoxyimidazoldion (6)

Die Reaktion ist in Fig. 2 dargestellt. Das 2-Methyl-2-Methoxyimidazoldion (6) kann durch Kondensation der Verbindung (7) mit Acetamidinhydrochlorid (8) in Gegenwart von Natriummethoxid in Methanol hergestellt werden. Die Nitrierung von 2-Methyl-2-Methoxyimidazoldion (6) erlaubt üblicherweise eine Gesamtausbeute von bis zu 38%. Nachteilig bei der Methode ist, dass das entstehende Zwischenprodukt (9) empfindlich ist und leicht explodiert und dass bei der Reaktion Reaktionswärme entsteht. Daher muss aus Sicherheitsgründen in großer Verdünnung gearbeitet werden. Dadurch sind verhältnismäßig große Reaktionsgefäße erforderlich, wodurch eine großtechnische Synthese unwirtschaftlich ist.

### 3. Nitrierung von 2-Methyl-4,6-pyrimidindion (10)

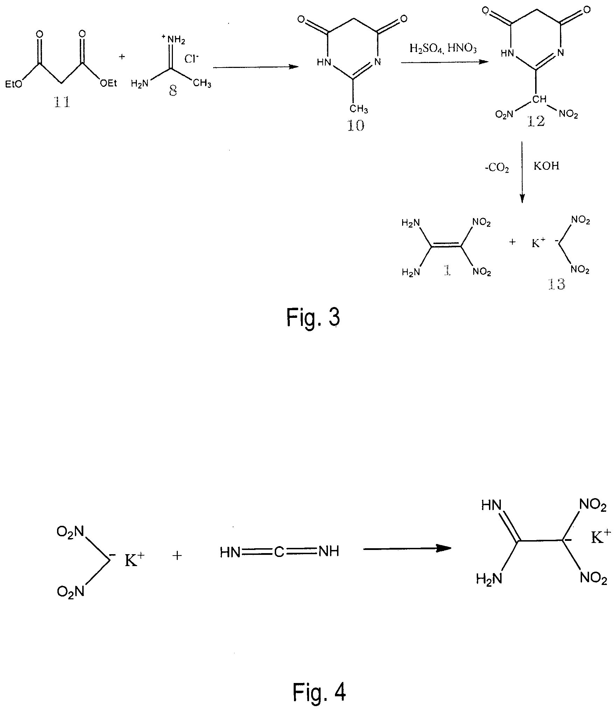

Das Verfahren ist in Fig. 3 dargestellt. 2-Methyl-4,6-pyrimidindion kann aus Diethylmalonat (11) und Acetamidinhydrochlorid (8) hergestellt werden. Die Ausbeute beträgt etwa 80%. Bei der Hydrolyse der nitrierten Zwischenverbindung (12) entsteht als Nebenprodukt Kaliumdinitromethanat (13). Da Kaliumdinitromethanat explosiv und temperaturempfindlich ist, ist es erforderlich, die Nitrierung langsam und temperaturkontrolliert durchzuführen. Dadurch ist die großtechnische Durchführung der Reaktion verhältnismäßig teuer. Nach Abschluss der Nitrierung wird das Kaliumdinitromethanat (13) durch Filtration abgetrennt.

### 4. Nitrierung von 4,6-Dihydroxy-2-methylpyrimidin

Aus Latypov, N. V. et al., Organic Process Research & Development, Bd. 11, Nr. 1, 2007, Seiten 56 bis 59 ist ein Verfahren zur Synthese von FOX-7 durch Nitrierung von 4,6-Dihydroxy-2-methylpyrimidin bekannt.

Da FOX-7 bisher verhältnismäßig teuer ist, findet es außer als Boosterladung für nukleare Gefechtsköpfe, Zündüberträger und Zündverstärker in Kleinmengen bisher kaum industrielle Anwendung. Wegen seiner hohen Leistung und geringen Sensitivität gegenüber Schlag, Reibung und thermischen Einflüssen wäre FOX-7 als Hochleistungssprengstoff für viele Anwendungen attraktiv, wenn es günstiger herzustellen wäre. Beispielsweise könnte es als insensitiver Leistungsverstärker in Scheinzielsätzen für spektrale Flares, als Leistungsverstärker in Treibsätzen sowie als Hauptladung in diversen Anwendungen eingesetzt werden. Aufgabe der vorliegenden Erfindung ist es, ein alternatives Verfahren zur günstigen Herstellung von FOX-7 oder eines Derivats davon bereitzustellen.

Die Aufgabe wird durch die Merkmale des Patentanspruchs 1 gelöst. Zweckmäßige Ausgestaltungen ergeben sich aus den Merkmalen der Patentansprüche 2 bis 8. Erfindungsgemäß ist ein Verfahren zur Synthese von 1,1-Diamino-2,2-dinitroethylen (FOX-7) oder eines Salzes von 1,1-Diamino-2,2-dinitroethylen vorgesehen, wobei ein Carbodiimid mit einem Dinitromethan-Anion umgesetzt wird. Das Umsetzen des Carbodiimids mit dem Dinitromethan-Anion erfolgt in einer Lösung. Es kann insbesondere in dem polaren aprotischen Lösungsmittel Dimethylformamid (DMF) erfolgen.

Bei dem Carbodiimid kann es sich insbesondere um C(NH)₂ handeln, welches mit Cyanamid in einem tautomeren Gleichgewicht vorliegt. Bei der Reaktion erfolgt eine nucleophile Addition an das Carbodiimid. Die Reaktion ist schematisch in Fig. 1 dargestellt. Bei der in Fig. 1 dargestellten Reaktion entsteht ein Salz von FOX-7. Das Salz lässt sich durch Ansäuern oder Neutralisieren der oder einer das Salz enthaltenden Lösung in FOX-7 überführen. Diese Synthese ist wesentlich kostengünstiger durchzuführen als die im Stand der Technik bekannte Synthese. Darüber hinaus ist sie wesentlich ungefährlicher, da dabei kein gefährlicher Nitrierungsschritt erforderlich ist und die Reaktion bei einer verhältnismäßig niedrigen Temperatur abläuft, bei der auch der Einsatz des an sich empfindlichen und explosiven Dinitromethan-Anions kein Sicherheitsproblem darstellt. Weiterhin läuft die Reaktion in Lösung ab und das Produkt kann aus der Lösung ausgefällt und abfiltriert werden. Die Ausgangsstoffe Carbodiimid und Dinitromethanat sind verhältnismäßig günstig.

Dem polaren aprotischen Lösungsmittel kann ein protisches Lösungsmittel zugesetzt werden. Bei dem protischen Lösungsmittel kann es sich um Wasser oder einen Alkohol, insbesondere einen Alkylalkohol, insbesondere Methanol, Ethanol oder Propanol, handeln. Das protische Lösungsmittel kann dabei den Ablauf der Reaktion begünstigende Wasserstoffatome liefern und dadurch die Reaktionsgeschwindigkeit erhöhen. Da Carbodiimid sehr leicht durch Hydrolyse oder Alkoholyse zerfällt, sollte nur eine verhältnismäßig geringe Menge des protischen Lösungsmittels zugesetzt werden. Das Zusetzen des protischen Lösungsmittels sollte erst am oder kurz nach Beginn der Reaktion erfolgen.

Bei einer Ausgestaltung des Verfahrens erfolgt das Umsetzen bei einem pH-Wert im Bereich von 6,5 bis 7,5. Dabei wird eine gute Ausbeute erzielt. Das Dinitromethan-Anion kann mit einem Kalium-Ion oder einem Ammonium-Ion als Gegenion assoziiert sein.

Ein bei der Synthese entstehendes 1,1-Diamino-2,2-dinitroethylen-Anion kann durch Verminderung des pH-Werts der/einer das 1,1-Diamino-2,2-dinitroethylen-Anion enthaltenden Lösung in 1,1-Diamino-2,2-dinitroethylen (FOX-7) überführt werden. Bei der Lösung kann es sich um die Lösung handeln, in welcher die Synthese erfolgt ist. Es ist jedoch auch möglich, dass das 1,1-Diamino-2,2-dinitroethylen-Anion in Form eines Salzes aus der Lösung, in welcher die Synthese erfolgt ist, abgesondert und dann im selben oder einem anderen Lösungsmittel gelöst wird, um die genannte Lösung zu bilden.

Nachfolgend wird die Erfindung anhand eines Ausführungsbeispiels näher erläutert.

Fig. 4 zeigt ein Reaktionsschema der Synthese von KFOX-7 aus Carbodiimid.

Zur Durchführung der in Fig. 4 dargestellten Reaktion werden in einem 50 ml Becherglass 778 mg (5,4 mmol) Kaliumdinitromethanat in 20 ml DMF bei 60 °C gelöst und 226 mg (5,4 mmol) Carbodiimid in Form von C(NH)₂ zugegeben. Die Lösung wird für 2 Stunden bei 60 °C gerührt und anschließend unter Rühren auf Raumtemperatur abkühlen gelassen. Nach Abdestillieren des Lösungsmittels wird der Rückstand bei 50 °C in einem explosionsgeschützten Trockenofen getrocknet und im Anschluss thermoanalytisch mittels dynamischer Differenzkalorimetrie (Differential Scanning Calorimetry, DSC) sowie IR-, NMR- und massenspektroskopisch vermessen. Die Ergebnisse sind in der nachfolgenden Tabelle 1 dargestellt.

**Tabelle 1**

| Analytik | Reaktionsprodukt | KFOX-7-Referenz |
|---|---|---|
| 1H-NMR (400 MHz) | 8,54 ppm (s) | 8,8 ppm (s) (FOX-7) |
| 13C-NMR (400 MHz) | 158,4 ppm (s) | 158,41 ppm (s) (KFOX-7) |
| Massenspektrum | 148,3 m/z | 148,1 m/z (FOX-7) |
| DSC | 230 °C | 228 °C (KFOX-7) |

Der Vergleich mit der KFOX-7-Referenz zeigt, dass das Reaktionsprodukt das 1,1-Diamino-2,2-dinitroethylen-Anion enthält.

Beim Ausführungsbeispiel kann statt des Kaliumdinitromethanats auch Ammoniumdinitromethanat und/oder statt C(NH)₂ auch ein anderes Carbodiimid eingesetzt werden.

## Patentansprüche

1. Verfahren zur Synthese von 1,1-Diamino-2,2-dinitroethylen (FOX-7) oder eines Salzes von 1,1-Diamino-2,2-dinitroethylen,
**dadurch gekennzeichnet,**
**dass** ein Carbodiimid mit einem Dinitromethan-Anion umgesetzt wird.

2. Verfahren nach Anspruch 1,
**dadurch gekennzeichnet,**
**dass** das Carbodiimid C(NH)₂ ist.

3. Verfahren nach Anspruch 1 oder 2,
**dadurch gekennzeichnet,**
**dass** Umsetzen des Carbodiimids mit dem Dinitromethan-Anion in einem polaren aprotischen Lösungsmittel erfolgt, wobei das polare aprotische Lösungsmittel Dimethylformamid (DMF) ist.

4. Verfahren nach Anspruch 3,
**dadurch gekennzeichnet,**
**dass** dem polaren aprotischen Lösungsmittel ein protisches Lösungsmittel zugesetzt wird.

5. Verfahren nach Anspruch 4, **dadurch gekennzeichnet,**
**dass** das protische Lösungsmittel Wasser oder ein Alkohol ist.

6. Verfahren nach einem der Ansprüche 3 bis 5,
**dadurch gekennzeichnet,**
**dass** das Umsetzen bei einem pH-Wert im Bereich von 6,5 bis 7,5 erfolgt.

7. Verfahren nach einem der vorhergehenden Ansprüche,
**dadurch gekennzeichnet,**
**dass** das Dinitromethan-Anion mit einem Kalium-Ion oder Ammonium-Ion als Gegenion assoziiert ist.

8. Verfahren nach einem der vorhergehenden Ansprüche,
**dadurch gekennzeichnet,**
**dass** ein bei der Synthese entstehendes 1,1-Diamino-2,2-dinitroethylen-Anion durch Verminderung des pH-Werts einer das 1,1-Diamino-2,2-dinitroethylen-Anion enthaltenden Lösung in 1,1-Diamino-2,2-dinitroethylen überführt wird.

## Claims

1. Process for the synthesis of 1,1-diamino-2,2-dinitroethylene (FOX-7) or a salt of 1,1-diamino-2,2-dinitroethylene,
**characterized in that**
a carbodiimide is reacted with a dinitromethane anion.

2. Process according to Claim 1,
**characterized in that**
the carbodiimide is C(NH)₂.

3. Process according to Claim 1 or 2,
**characterized in that**
the reaction of the carbodiimide with the dinitromethane anion is carried out in a polar aprotic solvent, wherein the polar aprotic solvent is dimethyl formamide (DMF).

4. Process according to Claim 3,
**characterized in that**
the polar aprotic solvent is admixed with a protic solvent.

5. Process according to Claim 4, **characterized in that** the protic solvent is water or an alcohol.

6. Process according to any of claims 3 to 5, **characterized in that**
the reaction is carried out at a pH in the range from 6.5 to 7.5.

7. Process according to any of the preceding claims,
**characterized in that**
the dinitromethane anion is associated with a potassium ion or ammonium ion as a counterion.

8. Process according to any of the preceding claims,
**characterized in that**
a 1,1-diamino-2,2-dinitroethylene anion formed in the synthesis is converted into 1,1-diamino-2,2-dinitroethylene by reduction of the pH of a solution containing the 1,1-diamino-2,2-dinitroethylene anion.

## Revendications

1. Procédé pour la synthèse de 1,1-diamino-2,2-dinitroéthylène (FOX-7) ou d'un sel de 1,1-diamino-2,2-dinitroéthylène,
**caractérisé en ce qu'**un carbodiimide est mis à réagir avec un anion du dinitrométhane.

2. Procédé selon la revendication 1,
**caractérisé en ce que** le carbodiimide est C(NH)₂.

3. Procédé selon la revendication 1 ou 2,
**caractérisé en ce que** la réaction du carbodiimide avec l'anion du dinitrométhane est mise en oeuvre dans un solvant polaire aprotique, le solvant polaire aprotique étant le diméthylformamide (DMF) .

4. Procédé selon la revendication 3,
**caractérisé en ce qu'**un solvant protique est ajouté au solvant polaire aprotique.

5. Procédé selon la revendication 4,
**caractérisé en ce que** le solvant protique est l'eau ou un alcool.

6. Procédé selon l'une quelconque des revendications 3 à 5,
**caractérisé en ce que** la réaction est mise en oeuvre à une valeur du pH dans la plage de 6,5 jusqu'à 7,5.

7. Procédé selon l'une quelconque des revendications précédentes,
**caractérisé en ce que** l'anion du dinitrométhane est associé à un ion potassium ou un ion ammonium en tant que contre-ion.

8. Procédé selon l'une quelconque des revendications précédentes,
**caractérisé en ce que** l'anion du 1,1-diamino-2,2-dinitroéthylène généré dans la synthèse est transformé en 1,1-diamino-2,2-dinitroéthylène par la réduction de la valeur de pH d'une solution contenant l'anion du 1,1-diamino-2,2-dinitroéthylène.
